# EUROPEAN PATENT APPLICATION

(11) **EP 1 738 713 A1**
(43) Date of publication of application: **03.01.2007**
(21) Application number: 05105807.1
(22) Date of filing: 29.06.2005
(51) Int. Cl.: A61C 1/14, A61B 17/16, B23B 51/12, B23B 31/20

(54) **Dental handpiece and kit including same**

(71) Applicant: Cohen, Yechiel, Carmiel 21861 (IL)
(72) Inventor: Cohen, Yechiel, Carmiel 21861 (IL)
(74) Representative: Weydert, Robert

(57) **Abstract**

A dental handpiece, adjustable in length, including a hand-gripping section at one end, and a tool-clamping section (6) at the opposite end; the tool-clamping section (6) including a sleeve (30) for receiving a shaft (8a) of a dental tool (8), and a collet for clamping the shaft (8a) of the dental tool (8) within the sleeve (30), the dental handpiece further comprising an extension piece (70) selectively receivable within the tool-clamping section (6) for extending the effective length of the dental tool (8); one end of the extension piece (70) including a shaft (71) receivable with the sleeve (30) of the tool-clamping section (6) for clamping therein by the collet; the opposite end of the extension piece (70) being formed with a sleeve for receiving the shaft (8a) of the dental tool (8), and having a clamping element (80) for clamping the shaft (8a) of the dental tool (8) within the sleeve of the extension piece (70).

## Description

### FIELD AND BACKGROUND OF THE INVENTION

The present invention relates to dental handpieces, and also to kits including such dental handpieces. The invention is particularly useful with respect to the dental handpiece described in Israel Patent 107202 of October 6, 1993, and corresponding U.S. Patents 5,549,474 of August 27, 1996 and 5,810,588 of September 22, 1998. The invention is therefore described below, for purposes of example only, with respect to such dental handpieces.

The above-cited Israel and U.S. Patents describe a dental handpiece comprising a housing including a hand-gripping section at one end, and a tool-clamping section at the opposite end. The tool-clamping section includes a sleeve for receiving a shaft of a dental tool, such as a drill or bur, and a collet for clamping the shaft of the dental tool within the sleeve. The dental handpiece further includes a button carried by the housing and depressible to cause the collet to release the shaft of the dental tool in order to permit convenient removable and replacement of the dental tool. The dental handpiece described therein is particularly useful for one including an air turbine for driving the dental tool at extremely high speeds, in the order of 400,000 rpm.

It has been found that the use of dental handpieces in general, and particularly the dental handpiece described in the above-cited patents, can be easily adapted for many different dental operation if the effective length of the dental tool can be increased in a quick and convenient manner. This is particularly true when the dental handpiece is used for preparing implants involving drilling holes of different depths into the patient's bone for receiving the implant.

### OBJECTS AND BRIEF SUMMARY OF THE PRESENT INVENTION

An object of the present invention is to provide a dental handpiece which permits the effective length of the dental tooth to be increased in a convenient manner, and thereby permits more convenient use of the dental handpiece for many different dental operations. Another object of the invention is to provide a kit including a dental handpiece and a plurality of extension pieces which may be selectively used for changing the effective length of the dental tool.

According to one aspect of the present invention, there is provided a dental handpiece, comprising: a housing including a hand-gripping section at one end, and a tool-clamping section at the opposite end; the tool-clamping section including a sleeve for receiving a shaft of a dental tool, and a collet for clamping the shaft of the dental tool within the sleeve; characterized in that the dental handpiece further comprises an extension piece selectively receivable within the tool-clamping section for extending the effective length of the dental tool; one end of the extension piece including a shaft receiveable with the sleeve of the tool-clamping section for clamping therein by the collet; the opposite end of the extension piece being formed with a sleeve for receiving the shaft of the dental tool, and having a clamping element for clamping the shaft of the dental tool within the sleeve of the extension piece.

According to further features in the preferred embodiment of the invention described below, the sleeve of the extension piece is integrally formed with the shaft of the extension piece. In addition, the sleeve of the extension piece is formed with an externally threaded section, and the clamping element includes an internally threaded section receivable on the externally threaded section of the sleeve for clamping the shaft of the dental tool within the sleeve of the extension piece.

According to still further features in the described preferred embodiment, the sleeve of the extension piece if further formed with a radially-contractible end section at the end thereof opposite to its shaft and formed with an external conical surface; the clamping element including an inner surface of complementary conical configuration engageable with the external conical surface of the end section for radially-contracting the end section to clamp the shaft of the tool therein when the internally-threaded section of clamping element is threaded on the externally-threaded section of the sleeve of the extension piece. In addition, the end section of the extension piece formed with the external conical surface is longitudinally split to facilitate the radial contraction of the end section. In addition, the sleeve of the extension piece is further formed with an externally ribbed surface at the end thereof joined to the shaft of the extension piece to facilitate manipulating the extension piece when applying it to the tool-clamping section of the housing, while the clamping element is formed with an outer ribbed surface to facilitate its application to the extension piece for clamping the shaft of the tool within the sleeve of the extension piece.

The invention is particularly useful in a dental piece including an air turbine for rotating the dental tool, e.g., a drill or bur, at high speed.

According to a further aspect of the invention, there is provided a dental kit including a dental handpiece as described above, the dental kit further including a plurality of extension pieces of different lengths selectively receivable within the tool-clamping section of the housing for extending the effective length of the dental tool clamped therein according to the extension piece selected.

As will be described more particularly below, the invention permits dental handpieces, particularly (but not exclusively) those driven by air tubines, to be more conveniently used in a wide variety of dental operation, especially in drilling holes for implants. In addition, providing such extension pieces of different length saves the substantial expense that would be involved in providing the various dental tools in different lengths.

Further features and advantages of the invention will be apparent from the description below.

### BRIEF DESCRIPTION OF THE DRAWINGS

The invention is herein described, by way of example only, with reference to the accompanying drawings, wherein:
Fig. 1 illustrates a prior art construction of a dental handpiece which the invention of the present application is particularly useful;
Fig. 2 is a side view, partly in section, illustrating an implementation of the present invention in the prior art dental handpiece of Fig. 1;
Fig. 3 is a three-dimensional view illustrating only the extension piece and the dental tool in the handpiece of Fig. 2;
Fig. 4 is a three-dimensional view illustrating the various elements of the extension piece and dental tool of Fig. 3 in an exploded condition; and
Figs. 5 and 6 illustrate extension pieces of different lengths that may be included in the kit for selective application to the dental tool in order to change its effective length as desired according to the particular dental operation to be performed with the dental tool.

It is to be understood that the foregoing drawings, and the description below, are provided primarily for purposes of facilitating understanding the conceptual aspects of the invention and various possible embodiments thereof, including what is presently considered to be a preferred embodiment. In the interest of clarity and brevity, no attempt is made to provide more details than necessary to enable one skilled in the art, using routine skill and design, to understand and practice the described invention. It is to be further understood that the embodiments described are for purposes of example only, and that the invention is capable of being embodied in other forms and applications than described herein.

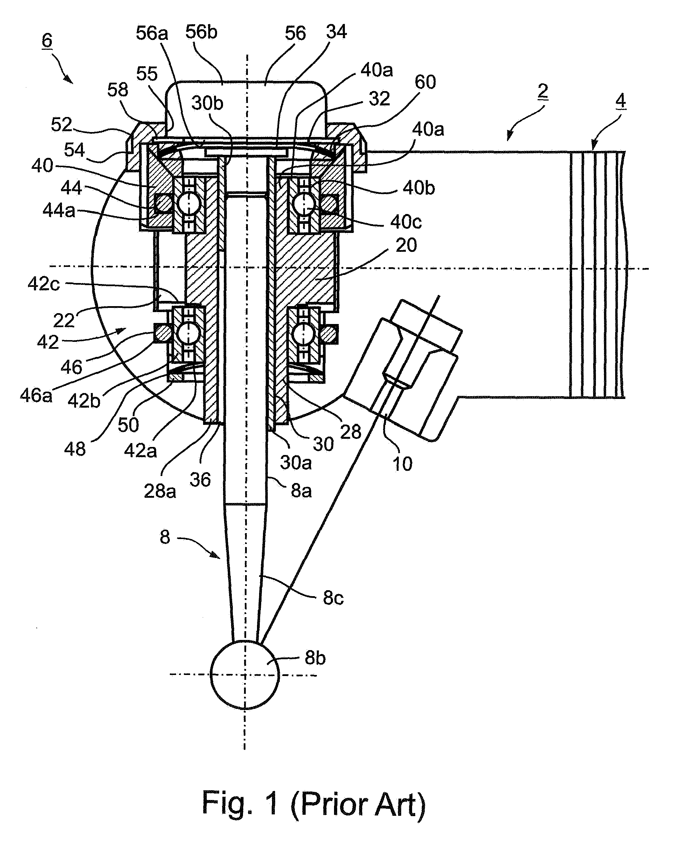

### DESCRIPTION OF A PRIOR ART DENTAL TOOL (FIG. 1)

As indicated earlier, the invention of the present application is particularly (but not exclusively) useful with the dental handpiece illustrated in the above-cited patents incorporated herein by reference. Fig. 1 illustrates the construction of such a prior art dental handpiece.

With reference first to Fig. 1, there is illustrated a dental handpiece including a housing, generally designated 2, having a hand-gripping section 4 at one end, and a tool-holding section 6 at the opposite end for holding a dental tool 8, such as a bur or drill. Section 6 of housing 2 is further formed with a water spray nozzle 10 for discharging a water spray onto the site being worked by the dental tool 8. The dental handpiece illustrated in Fig. 1 is an air turbine handpiece in which compressed air is supplied, via a conduit (not shown) at the back end of the handpiece, to an air turbine 20 within housing section 6 to rotate the dental tool 8 at a very high speed, e.g. in the order of 400,000 rpm. The rear end of the dental handpiece includes a further conduit (not shown) for introducing the water discharged in the form of a spray via nozzle 10 onto the working site of the dental tool.

For purposes of example, the dental tool 8 is shown as being of the bur type, including a shaft 8a of uniform diameter at one end, a bur 8b of spherical (or disc) shape at the opposite end, and a tapered juncture 8c between the shaft 8a and the bur 8b.

Air turbine 20 is formed with a plurality of blades 22 impinged by compressed air flowing from its inlet conduit to its outlet conduit (not shown) in order to rotate the air turbine at a very high speed. Air turbine 20 is integrally formed with a sleeve 28 of tapered configuration. One end of sleeve 28 is open, as shown at 28a, for receiving the shaft 8a of the dental tool 8. The inner diameter of tapered sleeve 28 decreases from its open end 28a to its opposite end 28b, which is also open.

Tapered sleeve 28 receives a tapered collet 30, which is also formed with an open end 30a received within the open end 28a of the tapered sleeve 28 for receiving the shaft 8a of the dental tool 8. The outer diameter of tapered collet 30 decreases from its open end 30a to its opposite end 30b. The latter end 30b is closed by an end wall or plug 32 formed within an enlarged head 34.

Tapered collet 30 is further formed with three axial slits 36 equally-spaced around its circumference. Slits 36 start at open end 30a and extend only for a part of the length of the collet towards the opposite end 30b. Slits 36 preferably extend about two-thirds the length of the tapered collet 30. They permit the respective section of the tapered collet to contract in order to firmly clamp therein the shaft 8a of the dental tool, or to expand in order to release the shaft for purposes of removing the dental tool or reinserting another dental tool in its place.

Two ball-bearing assemblies 40, 42, are provided at the opposite ends of housing section 6 for rotatably mounting the air turbine 20, its tapered sleeve 28, the tapered collet 30, and the dental tool 8 when clamped therein, with respect to the housing. Thus, each ball-bearing assembly 40, 42 includes: a rotary ring 40a, 42a; a static ring 40b, 42b; and a plurality of balls 40c, 42c interposed between both rings. Housing section 6 further includes a vibration-absorbing O-ring 44, 46, for each of the ball-bearing assemblies 40, 42, received within an annular recess 44a, 46a, and a spring 48 baring against a plastic ring 50 for preloading the static part of the bearing in order to reduce vibration during the highspeed rotation of the dental tool. A similar plastic ring 52 is provided at the opposite end of the housing section 6 cooperable with bearing assembly 40.

The latter end of housing section 6 is closed by a collar 54 threadedly attached to the housing section and formed with a central opening 55 in which a button 56 is mounted to project through the opening. Button 56 is formed with an outwardly-projecting rim 58 around its circumference engageable by the edge of collar 54 circumscribing opening 55 receiving the button. An annular spring 60 is interposed between the housing section 6 and button 56. Spring 60 is engageable within inner surface 56a of the button to urge the button outwardly, limited by the engagement of rim 58 of the button with the undersurface of collar 54. Pressing against the outer surface 56b of the button brings its inner surface 56a into engagement with the enlarged head 34 of the tapered collet 30, and thereby moves the tapered collet outwardly of the tapered sleeve 28. When this occurs, slits 36 formed in the outer section of the tapered collet 30 permit that section of the tapered collet, moving outwardly of the tapered sleeve 28, to expand and thereby to release the shaft 8a of the dental tool. The dental tool may then be manually grasped and removed from the tapered collet 30.

To insert another dental tool 8, button 56 is again depressed to move the tapered collet 30 outwardly of the tapered sleeve 28, and the dental tool is pushed inwardly into the tapered collet 30 until the end of the dental tool shaft 8a limits against the end wall 32 of the tapered collet. At that time, button 56 is released, whereupon spring 60 moves the button 56 slightly outwardly of the housing. The user then manually pushes the dental tool shaft 8a further inwardly into the housing. This also moves the tapered collet 30 further into the housing, until the enlarged head 34 of the tapered collet limits against the inner surface 56a of the button 56, at which time the dental tool 8 is firmly clamped in housing section 6 by the wedging action between the tapered collet 30 and the tapered sleeve 28.

Further details of the construction and operation of such dental handpieces are set forth in the above-cited patents, the contents of which are incorporated herein by reference.

### DESCRIPTION OF PREFERRED EMBODIMENTS OF THE PRESENT INVENTION

According to the present invention, the dental handpiece illustrated in Fig. 1 is adapted for many different dental operations, particularly in preparing implants, by providing one or more extension pieces for changing the effective length of the dental tool in a quick and convenient manner. Preferably, a plurality of such extension pieces are provided in different lengths in the form of a kit, each being selectively receivable within the tool-clamping section of the housing for extending the effective length of the dental tool clamped therein according to the extension piece selected. Such an arrangement not only adapts the dental handpiece for more convenient use in various dental operations, but also saves the substantial expense that would be involved if the various dental tools are provided in different lengths for the various dental operations.

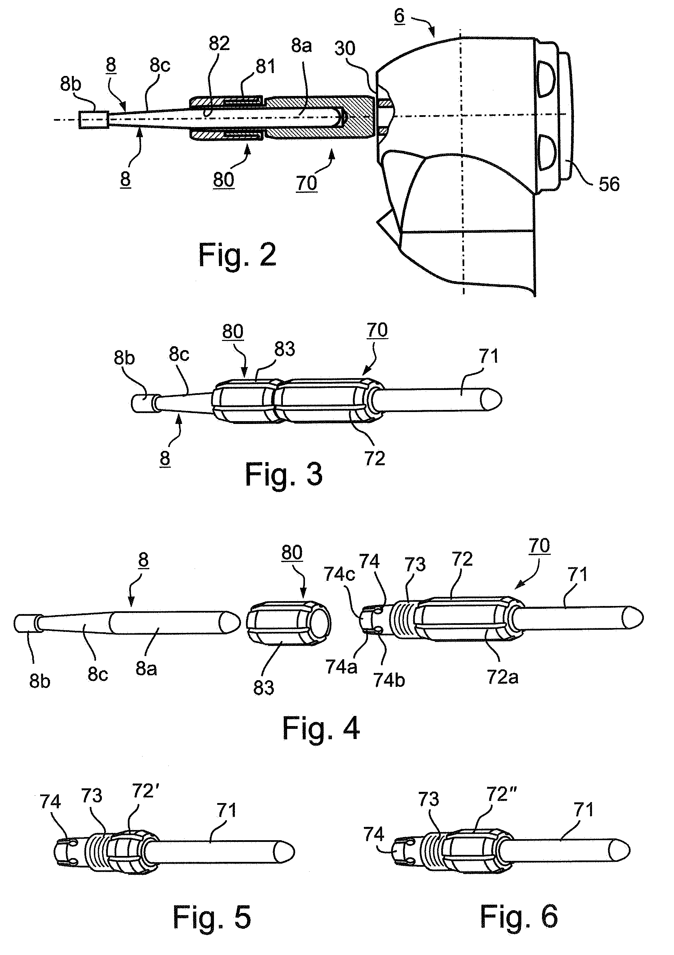

Fig. 2 illustrates an extension piece, therein generally designated 70, constructed in accordance with the present invention so as to be receivable within the tool-clamping section 6 of the dental handpiece for extending the effective length of the dental tool 8. Also shown in Fig. 2 is a clamping element, generally designated 80, for clamping the shaft 8a of the dental tool 8 to the extension piece 70.

Fig. 3 illustrates the extension piece 70, its clamping element 80, and the dental tool 8 in an assembled condition; whereas Fig. 4 illustrates the foregoing elements in an exploded condition.

As shown particularly in Fig. 4, extension piece 70 includes: a shaft 71 at one end receiveable within sleeve 30 of the tool-clamping section 6 of the dental handpiece, in lieu of shaft 8a of the dental tool 8; and a sleeve at the opposite end for receiving shaft 8a of the dental tool 8. The latter sleeve includes a finger-piece section 72 adjacent the shaft 71; an externally threaded section 73 for receiving the clamping element 80; and a radially-contractible end section 74, at the opposite end of extension piece 70 from its shaft 71, for engaging the outer surface of shaft 8a of the dental tool 8 and for clamping the dental tool within the extension piece 70.

Shaft 71 of extension piece 70 is of the appropriate diameter and length corresponding to shaft 8a of the dental tool 8 so as to be receivable within sleeve 30 of the tool-clamping section 6 of the dental handpiece in lieu of shaft 8a of the dental tool 8. Sleeve 72 is integrally formed with shaft 71 of the extension piece 70. Its finger-piece section is further formed with an externally ribbed surface, as shown at 72a, to facilitate manipulating the extension piece when applying it to the tool-clamping section 6 of the housing.

The radially-contractible end section 74 is split at its end by a plurality of longitudinally-extending slots 74a, each terminating in a circular opening 74b to increase the radial contractibility of the end section. The outer portion of end section 74 is formed with an outer conical surface 74c cooperable with a complementary conical surface within clamping element 80, to securely engage the dental tool shaft 8a, and to clamp it within the sleeve 70, when the clamping element 80 is applied to that end of the sleeve.

As shown particularly in Fig. 2, clamping element 80 is internally threaded at one end as shown at 81, for engagement with the external threads 73 of extension piece 70. The opposite end of clamping element 80 is formed with an internal conical surface, as shown at 82, for engagement with the external conical surface 74c of extension piece 70 when the clamping element is threaded onto the outer threaded surface 73 of the extension piece, in order to contract the end section 74 into firm engagement with the dental tool shaft 8a. As shown in Figs. 3 and 4, the outer surface of clamping element 80 is also formed with an outer ribbed surface 83 to facilitate application to the extension piece 70 for clamping the shaft 8a of the dental tool 8 within sleeve 72 of the extension piece.

The manner of using the extension piece 70 and clamping element 80 for changing the effective length of the dental tool 8 will be apparent from the above description.

Assume the dental tool 8 is clamped within the tool-clamping section 6 of the dental handpiece, and it is desired to change the effective length of the dental tool in order to enable it to be used for drilling a relatively deep hole in an implant operation. This may be done in the following manner:
First, button 56 is depressed to release shaft 8a of the dental tool 8 from collet 30 of the sleeve 28 within the tool-clamping section 6 of the dental handpiece, in the manner described above. With the button 56 depressed, shaft 71 of the extension piece 70 is inserted into the collet 30 of the tool-clamping section 6 of the dental handpiece, in the manner described above with respect to dental shaft 8a. The button is then released, thereby firmly clamping the extension piece 70 within the tool-clamping section 6 of the dental handpiece. The dental tool shaft 8a is then received within sleeve 72 of the extension piece 70 and moved to the end of the sleeve, as shown in Fig. 2. The clamping element 80 is then applied with its internally-threaded end 81 threaded over the externally-threaded section 73 of sleeve 72. This causes the internal conical surface 82 of the clamping element 80 to engage the external conical surface 74c of the end section 74 of the extension piece sleeve 72, to firmly clamp the dental tool shaft 8a within sleeve 72.

As indicated earlier, the invention is preferably included in a dental tool kit which includes a plurality of extension pieces 70 of different lengths to thereby enable the user to change the effective length of the dental tool by selecting the appropriate extension piece. Figs. 5 and 6 illustrate two such extension pieces which may be included in such a kit together with extension piece 70 described above with respect to Figs. 2-4. To change the effective length of the respective extension piece, only the length of the ribbed section 72 of the extension piece is changed. Thus, in the extension piece illustrated in Fig. 5, the ribbed section of the sleeve 72 is of substantially shorter length, as shown at 72', than in Fig. 4; whereas in the extension piece illustrated in Fig. 6, the ribbed section, shown at 72", is of longer length than in Fig. 5 but shorter than in Fig. 4.

It will be appreciated that the dental kit could be supplied with a smaller number or a larger number of extension pieces of different lengths to thereby enable the effective length of the dental tool to be varied as desired according to the particular dental operation to be performed with the dental handpiece.

While the invention has been described with respect to one preferred embodiment, it will be appreciated that this is set forth merely for purposes of example, and that many other variations, modifications and applications of the invention may be made.

## Claims

1. A dental handpiece, comprising: a housing including a hand-gripping section at one end, and a tool-clamping section at the opposite end; said tool-clamping section including a sleeve for receiving a shaft of a dental tool, and a collet for clamping the shaft of the dental tool within said sleeve;
**characterized in that** said dental handpiece further comprises an extension piece selectively receivable within said tool-clamping section for extending the effective length of said dental tool;
one end of said extension piece including a shaft receiveable with said sleeve of the tool-clamping section for clamping therein by said collet;
the opposite end of said extension piece being formed with a sleeve for receiving said shaft of the dental tool, and having a clamping element for clamping said shaft of the dental tool within said sleeve of the extension piece.

2. The dental handpiece according to Claim 1, wherein said sleeve of the extension piece is integrally formed with said shaft of the extension piece.

3. The dental handpiece according to Claim 1, wherein said sleeve of the extension piece is formed with an externally threaded section, and said clamping element includes an internally threaded section receivable on said externally threaded section of the sleeve for clamping the shaft of the dental tool within said sleeve of the extension piece.

4. The dental handpiece according to Claim 3, wherein said sleeve of the extension piece is further formed with a radially-contractible end section at the end thereof opposite to its shaft and formed with an external conical surface;
said clamping element including an inner surface of complementary conical configuration engageable with said external conical surface of said end section for radially-contracting said end section to clamp the shaft of the tool therein when the internally-threaded section of the clamping element is threaded on the externally-threaded section of the sleeve of the extension piece.

5. The dental handpiece according to Claim 4, wherein said end section of the extension piece formed with said external conical surface is longitudinally split to facilitate the radial contraction of said end section.

6. The dental handpiece according to Claim 4, wherein said sleeve of the extension piece is further formed with an externally ribbed surface at the end thereof joined to said shaft of the extension piece to facilitate manipulating the extension piece when applying it to said tool-clamping section of the housing.

7. The dental handpiece according to Claim 4, wherein said clamping element is formed with an outer ribbed surface to facilitate its application to the extension piece for clamping the shaft of the tool within the sleeve of the extension piece.

8. The dental handpiece according to Claim 1, wherein said housing further includes an air turbine for rotating said dental handpiece at high speed when the dental tool is received and clamped within said housing.

9. The dental handpiece according to Claim 1, wherein said housing further includes a button depressible to cause the collet to release the shaft of the dental tool, or of the extension piece, whichever is clamped therein by said collet.

10. A dental kit including a dental handpiece according to Claim 1, said dental kit further including a plurality of said extension pieces of different lengths selectively receivable within said tool-clamping section of the housing for extending the effective length of the dental tool to be clamped therein according to the extension piece selected.
